# EUROPEAN PATENT APPLICATION

(11) **EP 2 474 327 A1**
(43) Date of publication of application: **11.07.2012**
(21) Application number: 11150426.2
(22) Date of filing: 07.01.2011
(51) Int. Cl.: A61K 49/22, A61P 35/00, A61P 29/00, A61P 19/02

(54) **Microdosing of ultrasound contrast agents**

(71) Applicant: RWTH Aachen, 52062 Aachen (DE)
(72) Inventor: Palmowski, Moritz, 52072 Aachen (DE); Kießling, Fabian, 52074 Aachen (DE); Hauff, Peter, 10439 Berlin (DE); Bräutigam, 10629 Berlin (DE)
(74) Representative: Gerstein, Hans Joachim

(57) **Abstract**

The present invention relates to ultrasound contrast agent, like non-functionalised or functionalised microbubbles or non-functionalised or functionalised microparticles, for the use in diagnosis or as a pharmaceutical. In particular, the present invention relates to microdosing of functionalised microbubbles or functionalised microparticles for use in ultrasound diagnosis. In addition, the present invention relates to pharmaceutical or diagnostic compositions, in particular ultrasound contrast agents, containing functionalised microbubbles or functionalised microparticles at low dosage. Finally, the present invention relates to methods for the diagnosis, in particular, ultrasound diagnosis, and/or treatment of a subject using the functionalised microbubbles or functionalised microparticles in low dosage as defined herein.

## Description

### Microdosing of ultrasound contrast agents

The present invention relates to ultrasound contrast agents based on microbubbles or microparticles both in functionalised or non-functionalised form, for the use in diagnosis or as a pharmaceutical. In particular, the present invention relates to microdosing of microbubbles or microparticles both, functionalised or non-functionalised for use in ultrasound diagnosis. In addition, the present invention relates to pharmaceutical or diagnostic compositions, in particular ultrasound contrast agents, containing microbubbles or microparticles both, functionalised or non-functionalised, at low dosage. Finally, the present invention relates to methods for the diagnosis, in particular, ultrasound diagnosis, and/or treatment of a subject using the functionalised or non-functionalised microbubbles or microparticles in low dosage as defined herein.

### Background

The in vivo imaging of processes at the cellular and molecular level is an important part in better understanding physiological and pathophysiolocial procedures at the molecular level and offers new opportunities for diagnosing and treating oncological, cardiovascular and other diseases. Various techniques have been described allowing in vivo imaging. For example, positron emission tomography (PET) and SPECT (single photon emission computed tomography) represent well established molecular imaging technologies in clinical diagnostics by the detection of radioactively labelled probes. However, these techniques still have a low spatial resolution. Therefore, PET and SPECT are often combined with other imaging procedures, like CT or MRI. Thus, it is possible to combine anatomical with cellular or molecular or metabolic images providing high resolution imaging procedures. The suitability of molecular imaging has already been established at the clinical or research levels for other procedures, such as optical, magnetic and ultrasound imaging technologies. For ultrasound imaging technologies, ultrasound contrast agents (USCAs) have been described. These USCAs provide a contrast when used with ultrasonic waves. In this connection, contrast generally defines the intensity ratio of two neighbouring pixels, shown in the image replication. That is, the ultrasound imaging technologies rely on measuring the density and imaging the differences in density of structures of e.g., particular body compartments or tissues. To enhance the differences in density, the USCA have been developed.

Gas bubbles (microbubbles) have proven to be particularly advantages as USCA since gases process low acoustic impedance (i.e. low density) when compared with a surrounding tissue. The large differences in acoustic impedance between gases and tissue, the so called impedance jump, lead to an intensified reflection of acoustic waves at their interface.

Thus, the use of microbubbles and microparticles as USCA in diagnosis and therapy is generally accepted. That is, these types of USCA have been used widely in diagnostic applications in medical research and clinical practice. Generally, according to the current point of view, the various USCA formulations based on microbubbles or microparticles can be divided into six categories based on their morphology, how they function and their indication. These six categories are shown in table 1 below.

**Table 1: Organization of the USCA formulation into 6 categories.**

| **Category** | **Formulation** | **Characteristics** |
|---|---|---|
| 1 | Free gas bubble | Dissolves rapidly in blood and do not pass the capillary bed of the lung (right-heart CA) |
| 2 | Encapsulated gas bubble with a soft elastic lipid or protein shell (soft shell) | Stabilized gas bubble, which can pass the capillary bed of the lung (CA for the entire cardiovascular system) |
| 3 | Encapsulated soft shell gas bubble, whereby slightly soluble gases are used here (e.g. Perfluoro gases) | Gases with longer acoustic life span in the cardiovascular-system |
| 4 | Encapsulated gas bubble with a stable shell consisting of biodegradable polymers such as polylactide or cyanoacrylate (hard shell) | Gas bubbles with longer half live and specific acoustic characteristics such as Stimulated Acoustic Emission (SAE) |
| 5 | Encapsulated gas bubble with target-specific ligands on the shell surface | USCA for molecular imaging |
| 6 | Gas bubbles as carriers of therapeutic substances | Gas bubbles as drug delivery system (DDS) |

Typically, the USCAs are minuscule microbubbles with a size in the vicinity of 1 to 5 µm, which are stabilised with a thin shell. However, USCA including microbubbles and microparticles in the nano-type range have been described as well. In addition, various forms of USCAs are known in the art including the referenced soft and hard shell types, nano-sized and stabilised perfluorcarbon liquid drops which enlarge after ultrasound treatment only, thus acquiring their ultrasound contrast activity.

Further, ultrasound contrast agent includes microbubbles and microparticles having a specific type of electrostatic surface charge, having a specific chemical composition of the shell or consisting of different types of gas such as e.g., air, nitrogen, oxygen, carbon dioxide, inert gases, alkane, alkene, alkine or perfluoro-carbon-hydrogens or mixture of different type of gases.

For example, it is described in EP 0 921 807 that gas-mixtures containing hydrogen gas may be administered in liposomes, or gas-filled microparticles to patients. Administrations of liquids in microbubbles or microparticles are described in various documents, e.g. US 2003/0157023.

Microbubbles include aqueous suspensions in which the bubbles of gas are bounded at the gas/liquid interface by thin envelope involving a stabilised amphiphilic material disposed at the gas to liquid interface. Microbubbles suspensions are typically prepared by contacting powdered amphiphilic materials, e.g. freeze-dried preformed liposomes or freeze-dried or spray-dried phosphorlipids solutions, with air or other gas and then with an aqueous carrier, while agitating to generate a microbubble suspension which can then be administered, preferably shortly after its preparation. Examples of aqueous suspension of gas microbubbles and preparations thereof are disclosed for instance in US 5,271,928 or US 5,445,813. For example, gas-filled microparticles include suspensions in which the bubbles of gas are surrounded by a solid material envelope of a lipid or of natural or synthetic polymers. Typically, gas-filled microparticles have a nucleus-shell structure whereby the thickness of the shell or envelope may vary from a few nanometers to a few micrometers. Examples of gas-filled microparticles and of the preparation thereof are disclosed for instance in US 5,711,933 and US 6,333,021.

Microbubbles and microparticles, like gas microbubbles and gas-filled microparticles, are used both, in diagnostic and therapeutic approaches. That is, microbubbles and microparticles may represent potential drug carriers allowing target directed delivery of the drugs. This is particularly true for non-functionalised and functionalised microbubbles and microparticles. For example, functionalised gas-filled microparticles are described in US 2003/0157023. Herein, the gas-filled microparticles are functionalised by e.g., hydrolysing of a polyalkylcyanoacrylate shell. These gas-filled microparticles are particularly useful in ultrasound diagnosis.

Indeed, microbubbles and microparticles are used as contrast agents - in particular ultrasound contrast agents - since many years. A review about the currently available microbubbles and microparticles for use as ultrasound contrast agents is provided in Kiessling, F., et.al., 2009 Curr Med Chem, 16:627-42. Therein, an overview is provided about the development and state of the art of ultrasound contrast agents based on microparticles including soft-shell microbubbles and hard-shell microparticles. Moreover, said document identifies advantages and disadvantages of the different types of microparticles, in particular with respect to ultrasound diagnosis including stability and sensitivity.

Using functionalised microbubbles or microparticles provide the possibility of specifically binding or conjugating molecules or substances. Said molecules may be compounds allowing targeting of the microparticles influencing physical and mechanical properties of the microparticles or influencing the metabolism in the subject. That is, functionalisation allows altering the degradation and the metabolisation properties of the microbubbles and microparticles. Further, using functionalised microbubbles or microparticles modified by comprising a molecule or moiety conjugated or bound via the functionalised group allow targeting of said microbubbles or microparticles. USCA targeting of appropriate targets may be achieved by passive targeting or active targeting. Passive targeting is a rather non-specific target enhancement by microbubbles or microparticles, for example based on size, chemical properties and electrical charge of the shell as well as the type of encapsulated gas, the route of injection and by physiological processes of the immune system. In particular, the following principle mechanisms have been described for passive targeting: phagocytosis, interaction with the cell membranes and lymph flow transport.

Active targeting requires special molecular structures allowing targeting to a specific binding partners. Of course, this requires knowledge of the molecular targets within the living organism in certain physiological and pathophysiological situations and the possibility to select said special molecular structures allowing binding to the specific binding partners for later imaging. USCAs allowing active targeting are composed of a shell stabilised gas microbubble and a targeting moiety which may optionally linked by a linker moiety to the shell. The targeting moiety is connected with the surface of functionalised microbubbles or functionalised microparticles and, typically, the targeting molecular structures are ligands selected from antibodies, peptides, polysaccharides or aptamers. The coupling or linkage of the targeting moiety to the signalling moiety, namely, the functionalised microbubbles or functionalised microparticles may be achieved by direct or indirect connection. Direct connection means covalent coupling optionally via a linker molecule while indirect connection means coupling e.g. via the biotin-streptavidin system wherein no covalent linkage is present. However, for clinical application, the targeting moiety should be coupled preferably by covalent binding to the signalling moiety.

Molecular imaging with targeted ultrasound contrast agents of microbubbles or microparticles is possible. For example, molecular imaging of tumor angiogenesis using VEGFR-2 or lymph nodes using L-selectin are described (Hauff P. et al., Handbook of Exp. Pharmacology 2008 (185), 223-245). Hence, functionalised microbubbles or gas-filled microparticles, e.g. cyanoacrylate microbubbles conjugated to ligands including antibodies represent a useful tool for enhancing specificity and sensitivity of the ultrasound diagnosis.

However, according to the art, the microbubbles or microparticles commercially available (e,g, Definity®, Optison^{™} or SonoVue), when used as ultrasound contrast agents, are administered in amounts of approx. 2.5 x 10⁸ up to more than 10¹⁰ microbubbles or microparticles per patient to allow ultrasound diagnosis for identification, localisation and characterisation of diseases, disorders or conditions. These doses correspond with approx. 5mg up to 170mg solid matter per patient depending on the type of USCA. Therefore, the minimum recommended doses of all commercially available USCA are far above microdosing, hence, several adverse reactions are listed in the respective package leaflet.

Generally, microdosing is a technique for studying the behaviour of drugs in humans by the administration of doses so low that they are unlikely to produce whole-body effects, but high enough to allow monitoring the event to be studied. This allows evaluating the pharmacokinetics of new drug candidates with almost no risk of side effects. The dose of a new drug for human volunteers is typically about 100 times lower than the proposed e.g. therapeutic or diagnostic dosage (from approx. 1 to 100 micrograms per volunteer but not above).

As mentioned before, all commercially available USCA are fare above microdosing and have therefore the potential for adverse reactions in human. Therefore, there is a need to provide ultrasound contrast agents allowing microdosing to avoid adverse reactions while maintaining sufficient sensitivity. Furthermore, extensive work and moneys, required to obtain approval of new microparticles or microbubbles as medicinal products by the respective authorities (e.g., EMEA, FDA), can be dramatically reduced by USCA microdosing.

Thus, the object of the present invention is to provide compositions, in particular, functionalised or non-functionalised microbubbles and/or microparticles for use in diagnosis and therapy whereby said functionalised or non-functionalised microbubbles or said functionalised or non-functionalised microparticles allow detection or therapy with high sensitivity while reducing economic and medicinal drawbacks.

In particular, evaluation and approval of medicinal products becomes easier for receiving a marketing authorisation. Thus, the costs for developing an approved ultrasound contrast agent can be reduced significantly. In addition, the risk of adverse effects and allergic reactions may be reduced or substantially eliminated. Adverse effect may derive from vascular disruption induced by disintegrating microbubbles, microembolism and any kind of allergic reactions. These immunological reactions can reach from paresthesia, vomiting and headache to severe events such as anaphylactic shock. It must be kept in mind that all components of the USCA can be immunologic: the microbubble or microparticle itself, the linker and the targeting molecule. Hence, microdosing is favourable.

### Summary of the present invention

Hence, in a first aspect, the present invention relates to functionalised and non-functionalised microbubbles and/or functionalised or non-functionalised microparticles for the use in diagnosis, in particular, ultrasound diagnosis, or as a pharmaceutical characterised in that the functionalised or non-functionalised microbubbles or functionalised or non-functionalised microparticles are administered in concentrations below 100 µg solid material/70 kg body weight (<1.43 µg/kg body weight) of a human subject. Preferably, the functionalised and non-functionalised microbubbles or functionalised or non-functionalised microparticles are functionalised or non-functionalised polyalkylcyanoacrylates, like polybutylcyanoacrylates (PBCA).

In a preferred embodiment, said functionalised microparticles are coupled or conjugated with a diagnostic marker molecule, a targeting molecule or a therapeutic molecule. Also the Snap-Tag technology may be used to couple targeting units to the microbubbles or gas-filled microparticles. Alternative to direct coupling, different linkers may be used to connecting the targeting unit to the microbubbles or microparticles such as polyethylene-glycol (PEG). In another preferred embodiment, the functionalised microbubbles or microparticles are coupled with streptavidin or avidin. Said ligands (streptavidin or avidin) allow binding appropriate molecules having a biotin tag.

Moreover, the present invention relates to a method for the diagnosis, in particular, ultrasound diagnosis and treatment of a subject comprising the step of administering to a subject microbubbles or microparticles in a concentration of ≤ 100 µg/70 kg body weight. Finally, pharmaceutical or diagnostic compositions containing functionalised microbubbles and/or microparticles according to the present invention are useful for diagnosis, in particular, ultrasound diagnosis, as well as for the treatment of diseases, disorders or conditions.

The present inventors recognised that it is possible to administer functionalised, in particular, targeted ultrasound contrast agents in low dosage of < 100 µg/70 kg body weight

### Brief description of the drawings

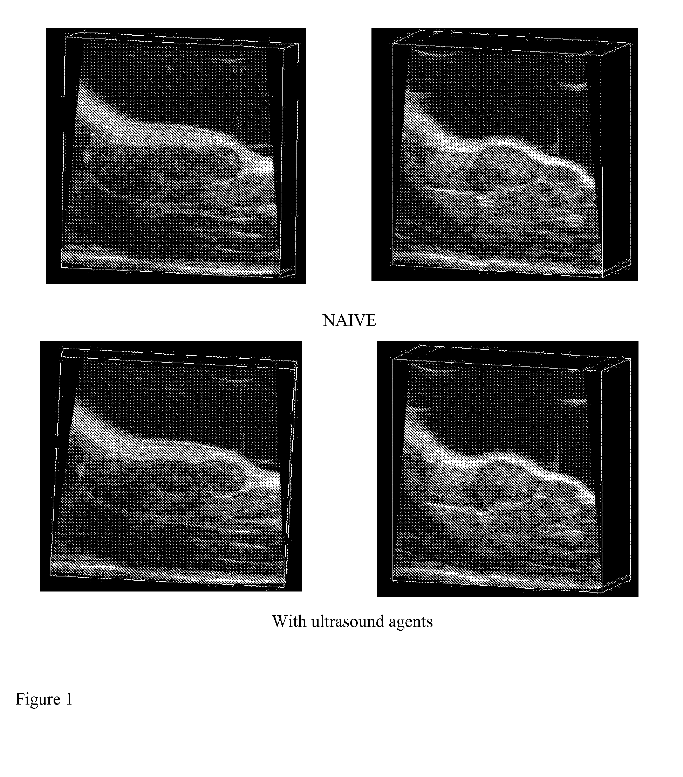

Figure 1: Shown are ultrasonic images by sensitive particle acoustic quantification (SPAQ) of tumors implanted subcutaneously. In the upper row images without a contrast agent are shown while the lower row show the same tumor using contrast agent which contain as a targeting moiety VEGFR2-specific antibody with microbubbles. Dosage is given as number of microbubbles per kg body weight. Single microbubbles can be identified within the tumor for both, higher microdosing and lower microdosing of microbubbles according to the present invention.

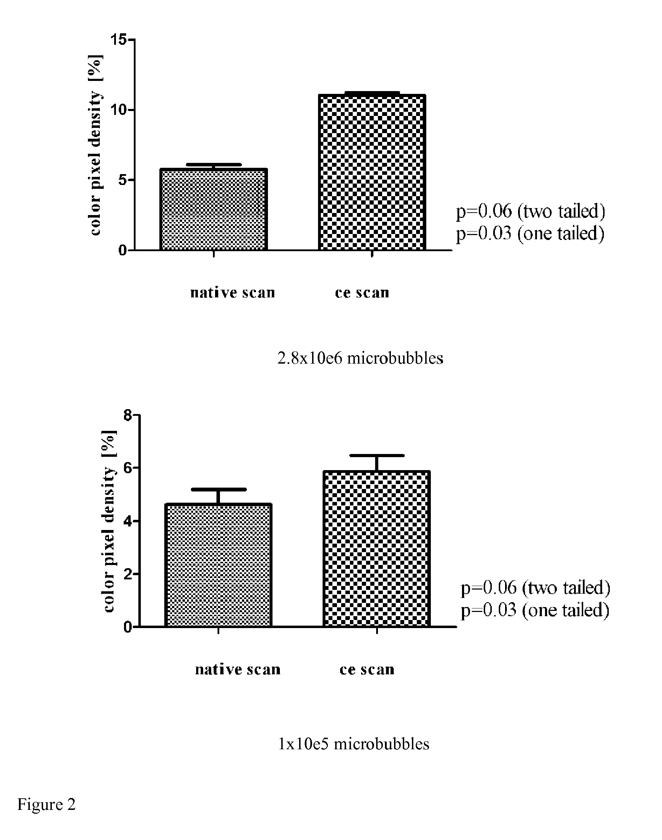

Figure 2 demonstrates that microdosing of microbubbles is sufficient to identify tumors. Even with microdosing of 1 x 10⁵ microbubbles/kg body weight corresponding to 2.40µg PBCA/70 kg body weight, statistically significant detection of contrast agent within the tumor is possible as it is the case for the higher microdosing of microbubbles shown in the upper graph (corresponding to 67.2 µg PBCA/70 kg body weight).

### Detailed description of the present invention

The present inventors recognised that it is possible to allow sensitive and specific ultrasound diagnosis using non- functionalised or functionalised microbubbles, or non- functionalised or functionalised microparticles when administering the same in a concentration of < 100 µg/70 kg body weight of a human subject.

This microdosing allows reducing adverse side effects including allergic reactions as well as having economic benefits including a simplified approval for medicinal products.

In the context of the present invention, the following terms are used:
Microparticles: A generic term for all particles and capsules containing gas or gas-forming substances (e.g. liquids) or other substances as cores which are stabilized by a so called hard shell and measuring between 500 nm and 500 µm, regardless of their structural design.
Microbubbles: A generic term for all a) bubbles, in particular, ultrasound contrast agents, consisting of gas or gas-forming substances (e.g. liquids) containing other substances or not which are non-stabilized or b) bubbles, in particular, ultrasound contrast agents, containing gas, gas forming substances or other substances as core which are stabilized by a so called soft shell, both measuring between 500 nm and 500 µm, regardless of their structural design.

It is preferred that the microparticles or microbubbles according to the present invention are gas-filled microparticles (GFMP) or gas-filled microbubbles.

The term "alkylcyanoacrylate" refers to an alkylester of cyanoacrylic acids. Polyalkylcyanoacrylate are polymers made of one or more alkylcyanoacrylates essentially without free acid and alcohol groups.

With the term "functionalised microbubbles" or "fuctionalised microparticles" is meant microbubbles or microparticles having functional groups. Basically, functionalisation refers to the presence of functionalised monomers and copolymers formed from said monomer whereby the functional groups are free for later reaction with other molecules or moieties conjugating the same with the microbubbles or microparticles. A functional group refers to a molecule group that contains at least one polar, reactive atomic compound with an X-H-group of atoms, with x = O, S and N. Functional monomers are monomers containing a functional group whereby said functional group may be a free functional group or a protected functional group. Protected functional group (non-functional group) refers to a functional group which is not available for reaction due to the presence of protective groups known in the art. A non-functional group can be converted into a functional group by cleavage of their protective group.

The term "microdosing" refers to a dosing of < 100 µg/70 kg body weight, corresponding to 1.43 µg/kg body weight.

The term "high dosage" refers to a dosage far above microdosing and is typically in the range between 5mg and 170mg per human for commercially available USCA.

The term "targeting moiety" refers to a compound or moiety allowing to specifically bind the microbubbles or microparticles, in particular, the functionalised microbubbles or functionalised microparticles, to cells, their epitopes or receptors (predominantly vascular or inflammation cells) or components of the interstitial matrix (such as fibronectin or collagen). This can be e.g. an antibody, a fragment of an antibody, a peptide, an aptamer, a lectin, nucleic acid or similar molecules. The term "targeting moiety" includes diagnostic marker molecules and therapeutic molecules.

The term "diagnostic marker molecule" refers to a compound or moiety allowing diagnosis of a disease, disorder or condition.

The term "therapeutic molecule" refers to a compound or moiety allowing to treat diseases by specifically binding to a structure in vivo. This can be an antibody, a fragment of an antibody, a peptide, an aptamer, a lectin, nucleic acids or similar molecules. The therapeutic molecule may also be used for diagnosis in lower dosages. Combined diagnostic and therapeutic compounds (which can also include drug loaded microbubbles) are called diagnostic agents.

It was recognised that micordosing of ultrasound contrast agent based on non- functionalised or functionalised microbubbles or non- functionalised or functionalised microparticles minimises the risk of adverse side effects and allergic reactions as well as enables reducing the costs for approval for medicinal products while maintaining the desired sensitivity of diagnosis.

It is particularly preferred that the non- functionalised or functionalised microbubbles or non- functionalised or functionalised microparticles are administered in a dosage of functionalised microbubbles or gas-filled microparticles of < 100 µg/70 kg body weight.

Said functionalised microbubbles or microparticles are preferably capsules comprising functionalised polyalkylcyanoacrylates or polylactic acids. Functionalised polyalcylcyanoacrylates are described e.g. in US 2003/0157023 A1 which is incorporated herein fully by reference. That is, the functionalised microparticles or functionalised microbubbles comprise functionalised polyalkylcyanoacrylates optionally in combination with non-functionalised polyalkylcyanoacrylates. In particular, the functionalised microparticles or functionalised microbubbles are gas-filled microparticles or functionalised microbubbles containing functionalised polyalkylcyanoacrylates. The functionalised polyalkylcyanoacrylate can be produced by a copolymerization of one or more alkylcyanoacrylates preferably butyl-, ethyl- and/or isopropylcyanoacrylate with a functional monomer, preferably, cyanoacrylic acid, and/or by a partial side-chain hydrolysis of a polyalkylcyanoacrylate, preferably polybutyl-, polyethyl- and/or polyisopropylcyanoacrylate. Suitable functional monomers are: Cyanoacrylic acid (H₂C=C(CN)-CO-OH), methacrylic acid (H₂C=C(CH₃)-CO-OH), methylenemalonic acid (H₂C=C(CO-OH)₂) and α-cyanosorbic acid (H₃C-CH=CH-CH=C(CN)-CO-OH) and their derivatives with the general formulas:
H₂C=C(CN)-CO-X-Z (cyanoacrylic acid derivatives),
H₂C=C(CH₃)-CO-X-Z (methacrylic acid derivatives),
H₂C=C(CO-X'-Z')₂ (methylenemalonic acid derivatives) and
H₃C-CH=CH-CH=C(CN) -CO-X-Z (α-cyanosorbic acid derivatives) with
X = -O-, -NH- or -NR¹- and
Z = -H, -R²-NH₂, -R²-NH-R¹, -R²-SH,R²-OH, R²-C(NH₂)-R¹ 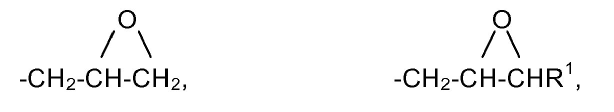 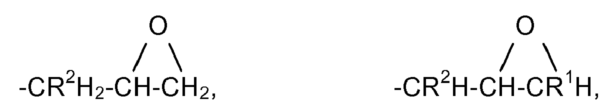
whereby R¹ = linear or branched alkyl radical and R² = linear or branched alkylene radical with respectively 1 up to 20 carbon atoms, and whereby both X' and Z', in each case independently of one another, have the meaning that is indicated for X and Z.

Substituted styrenes (Y-C₆H₄-CH=CH₂) or methylstyrenes (Y-C₆H₄-C(CH₃)=CH₂) with Y = -NH₂, -NR¹H, -OH, -SH, -R²-NH₂, -R²-NH-R<1>, -R²-SH, R²-OH, -R²-HC (NH₂)-R¹, whereby R¹ = linear or branched alkyl radical and R² = linear or branched alkylene radical with respectively 1 up to 20 carbon atoms.

Alternative polymeric substances or their monomeric precursors may be a) homopolymers such as polyethylene, polypropylene, polyvinylchloride and polyamide; b) copolymers such as polyester, polyurethane and some polyamide; c) natural biopolymers such as polysaccharides, proteins and peptides, and polynucleotide; d) bio-based polymers such as polylactids or polyhydroxybutyrats; e) synthetic biopolymers such as polyesters, polyvinyl-alcohols, polybutylenadipatterephthalat, polybutylensuccinat, polycaprolactone, polyglycolids or f) synthetic polymers such as polyethylene, polystyrole or polyvinylchloride. Furthermore, silane containing compounds or denaturized proteins are also included.

It is preferred that the functionalised microbubbles or functionalised microparticles are conjugated with a targeting moiety (e.g. a diagnostic marker molecule or a therapeutic molecule) which is conjugated to the functionalised microbubbles or functionalised microparticles by coupling the same via the functionalised functional group. For example, the coupling of the targeting moiety, like a diagnostic marker molecule or a therapeutic molecule may be affected via carboxylic acid groups as described in Kiessling F., et. al., 2009, Current Medicinial Chemistry, 16(5), 627-642.

In another embodiment, the functionalised microbubbles or functionalised microparticles are conjugated with streptavidin or avidin. Said streptavidin or avidin conjugated functionalised microbubbles or functionalised microparticles allows to non-covalently attach further molecules, including diagnostic marker molecules, therapeutic molecules or targeting molecules having a biotin moiety. Hence, a non-covalent binding system of functionalised microbubbles or functionalised microparticles is provided.

In the art, conjugation of the VEGFR-2 receptor to functionalised microbubbles or functionalised microparticles is described. Further, integrin targeting moieties may be conjugated as well as other known ligands of cell receptors. Furthermore, monoclonal or polyclonal antibodies may be conjugated with the functionalised microbubbles or functionalised microparticles for use in diagnosis or therapy.

In addition, the functionalised microparticles or functionalised microbubbles itself are stabilised by functionalisation, thus, enabling improved detection properties in ultrasound diagnosis.

A further aspect of the present invention relates to a method for diagnosis, in particular, ultrasound diagnosis, or treatment of a subject comprising the step of administering to a subject non-functionalised or, preferably, functionalised microbubbles, or non-functionalised or, preferably, functionalised microparticles in a concentration of < 100 µg/70 kg body weight, in particular, said method comprises the administration of the non-functionalised or, preferably, functionalised microbubbles or non-functionalised or, preferably, functionalised microparticles according to the present invention. The method relates in particular to ultrasound diagnosis, for example ultrasound diagnosis of disorders, diseases or conditions like angiogenesis, vascular inflammation, atherosclerosis, or ischemia. Another important field where these microbubbles can be used is therapy response monitoring.

In addition, the present invention relates to a pharmaceutical or diagnostic composition containing non-functionalised or functionalised microbubbles and/or non-functionalised or functionalised microparticles according to the present invention. The pharmaceutical or diagnostic composition may contain additional components typically present in pharmaceutical or diagnostic compositions.

For example, suitable liquid carriers are water, aqueous solutions such as saline or solutions of one or more tonicity adjusting substances. Storage of the pharmaceutical or diagnostic composition according to the present invention is possible in the freeze-dried form or in suspension whereby the freeze-dried form allows storage for a relatively long period of time before reconstitution. Administration of the functional microbubbles and/or functional microparticles according to the present invention as a pharmaceutical or diagnostic composition may be affected in various ways depending on its specific formulation. Examples of suitable administrations are, for instance, intravasculary, intralym-phatically, parenterally, subcutaneously, intramuscularly, intradermally, intraperitoneally, interstitially, intraarticulary, intranasaly, orally, or topically using a variety of dosage forms. Typically, ultrasound contrast agents are administered by intravenous injection or by injection into the urinary bladder.

The functionalised microbubbles and/or functionalised microparticles according to the present invention administered in microdosing allows to reach vascular targets, like endothelium in vessels, but also areas in the tissue having an altered permeability, like tumors or a side of inflammation, but also intestinal targets.

The methods according to the present invention using the microdosing of functionalised microbubbles or functionalised microparticles allow e.g. for characterising angiogenesis and inflammation in tumors, atherosclerosis and rheumatoid diseases. Further, the use of microdosing is particularly useful on all interventional applications, e.g. interventional coronary angiography. It is possible to differentiate plaques etc. Even repeated diagnosis is possible since the whole body dosage of the repeated microdosing is below the amount of ordinary high dosage diagnosis or treatment.

In the following, the invention will be described in more detail by reference to examples. The examples are provided for illustrating the claimed matter without limiting the same.

### Example 1

Air-filled polybutylcyanoacrylate microparticles (PBCA-MPs) were prepared following the one step method described in Hauff P. et.al., Radiologie 2004, 231: 667-673. Alternatively, PBCA-MPs were prepared according to the two-step method described in Schmidt & Rössling, Chemical Engineering Science, 2006, 4973-4981.

The PBCA-MPs of both preparation methods were functionalised by adding NaOH to the PBCA-MP leading to a carboxylation (hydrolysation) of the PBCA-MP shell surfaces. Streptavidin is covalently bound to the functionalised PBCA carboxylated gas-filled microparticles via the carboxylate group in the following referred to as carboxy-PBCA-MP. By adding biotinylated antibodies, effective coupling of the antibody to the microparticle surface is possible, thus, obtaining target specific PBCA microparticles for molecular imaging as described in Hauff P. et. al., Radiology, 2004, 231:667-673.

Due to the high acoustic sensitivity single particle molecular imaging with ultrasound is possible. In the present case, the method of sensitive particle acoustic quantification (SPAQ) is applied. Details of said technology are identified in Reinhard et. al., 2005, Invest Radiol, 40(1):2-7 and Palmowski et. al., 2008, Mol Cancer Ther, 7(1):101-109.

### Example 2

### Quantification of the amount of polymer for defined amounts of PBCA-MP

For determining the amount of PBCA per MP in vitro analytic was performed. In a first step, a calibration curve with pure PBCA-polymer was obtained. For preparing said PBCA-polymers, 3 ml BCA-monomer were added dropwise to 20 ml water under stirring conditions. Subsequently, the polymer was dried. The amount of polymer was determined with FT-IR spectrometry whereby the polymer was dissolved in tetrahydrofuran (THF) and the calibration curve was obtained.

Thereafter, hydrolysed samples of the PBCA-MP charges obtained in example 1 were prepared having concentrations of 1 x 10⁹ and 1 x 10¹⁰ MP/ml, respectively, dried and the weight was determined. After determining the weight, the samples were dissolved in THF and analysed spectrometrically by FT-IR spectrometry. Data are given in the table below.

| | MP/ml | *FT-IR value* | PBCA concentration mg/ml |
|---|---|---|---|
| After example 1 | 1 x 10⁹ | 0.1793 | 0.4359 |
| After example 1 | 1 x 10¹⁰ | 1.5024 | 3.4302 |
| After example 2 | 1 x 10⁹ | 0.2125 | 0.5111 |
| After example 2 | 1 x 10¹⁰ | 1.9507 | 4.4448 |

### Example 3

### Microdosing in vivo of tumor bearing mice

Target specific PBCA-MP were prepared according to claim 1, functionalised and conjugated with streptavidin as described. Biotinylated antibodies against the vascular endothelial growth factor receptor type 2 (VEGFR2) were attached to the streptavidin modified PBCA-MP. Six mice bearing a tumor of 5 mm in size developed after treating said mice with human epidermoid carcinoma cells (A431 cells) were used for further examination. For determining the targeted PBCA-MP quantitatively in vivo dosing of 2.8 x 10⁶ / kg bodyweight (bw) (corresponding to 0.96 µg PBCA / kg bw) and 1 x 10⁵ / kg bw (corresponding to 0.034302 µg PBCA / kg bw) were analysed. As shown in figures 1 and 2, it is possible to determine quantitatively PBCA-MP targeted to the tumor within the tumor of the respective mice.

Hence, microdosing of non-functionalised and, in particular, functionalised microbubbles, or non-functionalised and, in particular, functionalised microparticles for ultrasound diagnosis is possible.

## Claims

1. Non-functionalised or functionalised microbubbles or non-functionalised or functionalised microparticles for use in diagnosis, in particular, ultrasound diagnosis, or as a pharmaceutical **characterised in that** the non-functionalised or functionalised microbubbles or non-functionalised or functionalised microparticles are administered in concentrations of < 100 µg/70 kg body weight of a human subject.

2. The microbubbles or microparticles according to claim 1 in form of functionalised microbubbles or functionalised microparticles.

3. The microbubbles or microparticles according to claim 1 or 2 **characterized in that** the microbubbles or microparticles comprises polyalkylcyanoacrylates; homopolymers, in particular, polyethylene, polypropylene, polystyrole, polyvinylchloride and/or polyamide; copolymers, in particular, polyester, polyurethane and/or polyamide; natural biopolymers, in particular, polysaccharides, proteins and peptides, and/or polynucleotides; biopolymers, in particular, polylactids or polyhydroxybutyrats; and/or synthetic biopolymers, in particular, polyesters, polyvinyl-alcohols, polybutylenadipat-terephthalat, polybutylensuccinat, polycaprolactone, polyglycolids.

4. The functionalised microbubbles or funtionalised microparticles according to claim 2 or 3 **characterised in that** the microbubbles or microparticles comprises functionalised polyalkylcyanoacrylates, preferably functionalised polybutylcyanoacrylates.

5. The functionalised microbubbles or functionalised microparticles according to claim 4 wherein the functionalised polyalkylcyanoacrylate is produced by a copolymerisation of one or more alkylcyanoacrylates with a functional monomer.

6. The functionalised microbubbles or funtionalised microparticles according to any one of the preceding claims whereby the functionalised microbubbles or functionalised microparticles are conjugated with a targeting moiety, e.g. diagnostic marker molecule or a therapeutic molecule.

7. The functionalised microbubbles or functionalised microparticles according to any one of the preceding claims wherein the functionalised microbubbles or functionalised microparticles are conjugated with another diagnostic agent for hybrid imaging.

8. The functionalised microbubbles or functionalised microparticles according to any one of the preceding claims wherein the functionalised microbubbles or functionalised microparticles are conjugated with streptavidin, avidin, SNAP-tag or PEG.

9. The functionalised microbubbles or functionalised microparticles according to any one of the preceding claims wherein the functionalised microbubbles or functionalised microparticles are conjugated with a targeting molecule.

10. The functionalised microbubbles or microparticles according to claim 6 or 9 whereby the targeting moiety is a tumor specific molecule, in particular, VEGFR2, endoglin, integrins, selectins, aminopeptidases.

11. A method for the diagnosis, in particular, ultrasound diagnosis, and therapy monitoring of a subject comprising the step of administering to a subject microbubbles or microparticles in a concentration of < 100 µg/70 kg body weight.

12. A method for diagnosing or treating a subject comprising the step of administering functionalised microbubbles or functionalised microparticles according to any one of claims 1 to 10.

13. The method according to claims 11 or 12 for the ultrasound diagnosis of cancer, atherosclerosis, inflammation e.g. bowel or arthritis and for monitoring therapy response.

14. Pharmaceutical or diagnostic composition containing functionalised microbubbles and/or functional microparticles according to any one of claims 1 to 10.

15. The non-functionalised or functionalised microbubbles or non-functionalised or functionalised microparticles according to any one of claims 1 to 10 for use in the characterisation of angiogenesis and inflammation in tumors, atherosclerosis and rheumatoid diseases and for use in interventional applications.
